(19) Europäisches Patentamt
European Patent Office
Office européen des brevets

(11) **EP 4 303 586 A1**

(12) **EUROPEAN PATENT APPLICATION**
published in accordance with Art. 153(4) EPC

(43) Date of publication:
**10.01.2024  Bulletin 2024/02**

(21) Application number: **22763431.8**

(22) Date of filing: **04.03.2022**

(51) International Patent Classification (IPC):
***G01N 33/86*** (2006.01)

(52) Cooperative Patent Classification (CPC):
**G01N 33/86**

(86) International application number:
**PCT/JP2022/009429**

(87) International publication number:
**WO 2022/186381 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **05.03.2021   JP 2021035446**

(71) Applicant: **Sekisui Medical Co., Ltd.**
**Tokyo 103-0027 (JP)**

(72) Inventors:
• **KAWABE, Toshiki**
  **Tokyo 103-0027 (JP)**
• **ODA, Yukio**
  **Tokyo 103-0027 (JP)**

(74) Representative: **Wächtershäuser & Hartz**
**Patentanwaltspartnerschaft mbB**
**Weinstraße 8**
**80333 München (DE)**

(54) **METHOD FOR ESTIMATING FACTOR OF PROLONGED COAGULATION TIME**

(57)   A method for estimating a cause of coagulation time prolongation includes 1) detecting a coagulation reaction end point Pe in a coagulation reaction curve of a subject blood specimen having a prolonged coagulation time; 2) calculating T(X), wherein T(X) represents a measurement point or time at which the coagulation reaction curve reaches X% of Pe, and X is a variable of greater than 0 and equal to or less than 100; and 3) estimating a cause of coagulation time prolongation of the subject blood specimen based on a form of T(X).

Fig. 2

EP 4 303 586 A1

**Description**

Field of the Invention

The present invention relates to a method for estimating a cause of coagulation time prolongation.

Background of the Invention

A blood coagulation test is a test for diagnosing a blood coagulation ability of a patient by adding a predetermined reagent to a blood specimen of the patient and measuring blood coagulation time or the like. Typical examples of the blood coagulation time include prothrombin time (PT), activated partial thromboplastin time (APTT), and thrombin time. Abnormality in blood coagulation ability causes prolongation of coagulation time. Examples of the cause of prolongation of coagulation time include influence of coagulation inhibitors, a decrease of components involved in coagulation, a congenital blood coagulation factor deficiency, and an acquired appearance of autoantibody that inhibits coagulation reaction.

Conventionally, when prolongation of coagulation time is observed in a blood coagulation test and, in general, when there is no suspicion of administration or contamination of heparin to the specimen, a cross mixing test is performed to determine a cause of coagulation time prolongation. In the cross mixing test, the APTT (immediate reaction) immediately after the mixing of a blood specimen and a normal specimen and the APTT (late reaction) after incubation at 37°C for 2 hours are measured, and based on the patterns of the immediate reaction and late reaction, it is determined which of a coagulation factor inhibitor (anticoagulant), a lupus anticoagulant (LA), and a coagulant factor deficiency such as hemophilia causes the prolongation cause of the APTT. However, the cross mixing test is very laborious and time-consuming as described above. When the prolongation cause of coagulation time is heparin, the cross mixing test cannot determine the prolongation cause.

Patent Literature 1 describes that whether test plasma is suspected to be coagulation factor-deficient plasma is determined based on the value of a parameter related to differentiation of coagulation waveform of a plasma mixture of the test plasma and normal plasma and that abnormality other than coagulation factor deficiency in the test plasma, such as the presence of a coagulation factor inhibitor, the presence of LA, and the presence of an agent that affects blood coagulation, is investigated. However, the determination disclosed in Patent Literature 1 demands for preparation of a plasma mixture. In addition, Patent Literature 1 does not disclose a specific method for determining the presence of an agent that affects blood coagulation, such as heparin.

Patent Literature 2 discloses a method for determining whether a blood specimen is suspected to be a specimen derived from a subject having lupus anticoagulant (LA) or a coagulation factor inhibitor based on the value of a parameter related to differentiation of coagulation waveform of the blood specimen. However, the method disclosed in Patent Literature 2 determines the presence of LA or a coagulation factor inhibitor and also demands for differentiation of a coagulation waveform (coagulation reaction curve) for the determination.

As described above, in known technologies including the cross mixing test, no technology for determining the presence of heparin in a blood specimen from a coagulation waveform has been established. In addition, no technology of detecting coagulation factor deficiency without using a plasma mixture has been disclosed.

Patent Literature 3 describes that the time for coagulation reaction to reach a predetermined percentage of the maximum value of a coagulation reaction curve, the maximum value of a coagulation reaction rate curve and the time for reaching the maximum value, and each parameter related to the center of gravity point are related to the concentration of the blood coagulation factor.

Citation List

Patent Literature

    Patent Literature 1: JP-A-2016-194426
    Patent Literature 2: JP-A-2016-118442
    Patent Literature 3: WO 2020/101025

Summary of the Invention

The present invention provides an easier method for estimating a cause of coagulation time prolongation of a blood specimen.

That is, the present invention provides the followings.

[1] A method for estimating a cause of coagulation time prolongation, comprising:

1) detecting a coagulation reaction end point Pe in a coagulation reaction curve of a subject blood specimen having a prolonged coagulation time;
2) calculating T(X), wherein T(X) represents a measurement point or time at which the coagulation reaction curve reaches X% of Pe, and X is a variable of greater than 0 and equal to or less than 100; and
3) estimating a cause of coagulation time prolongation of the subject blood specimen based on a form of T(X).

[2] The method according to [1], wherein the 3) comprises calculating an indicator S representing the form of T(X) and estimating a cause of coagulation time prolongation of the subject blood specimen based on a value of the indicator S.
[3] The method according to [2], wherein the indicator S is an indicator representing a relative value of T(X) or a change rate of T(X).
[4] The method according to [3], wherein

the 3) further comprises estimating a cause of coagulation time prolongation of the subject blood specimen by comparing the indicator S with a reference value, and
the reference value is determined based on an indicator S calculated from a blood specimen group in which the cause of coagulation time prolongation is known.

[5] The method according to [3], wherein

the 3) further comprises estimating the subject blood specimen to be heparin positive when the indicator S is less than threshold 1 or when the indicator S is equal to or less than threshold 1,
wherein the thresholds 1 and 2 are reference values determined based on indicator S calculated from a blood specimen group including lupus anticoagulant positive.

[6] The method according to [3], wherein

the 3) further comprises estimating the subject blood specimen to be lupus anticoagulant positive when the indicator S is equal to or greater than threshold 1 and equal to or less than threshold 2 or when the indicator S is greater than threshold 1 and less than threshold 2,
wherein the thresholds 1 and 2 are reference values each determined based on indicator S calculated from a blood specimen group including lupus anticoagulant positive.

[7] The method according to [3], wherein

the 3) further comprises estimating the subject blood specimen to be coagulation factor deficiency or coagulation factor inhibitor positive when the indicator S is greater than threshold 2 or when the indicator S is equal to or greater than threshold 2,
wherein the threshold 2 is a reference value determined based on indicator S calculated from a blood specimen group including lupus anticoagulant positive.

[8] The method according to any one of [5] to [7], wherein the indicator S is $[T(X_1)/T(X_2)]$, (wherein $X_1 > X_2$, $X_1 = 30$ to 85, and $X_2 = 5$ to 20).
[9] The method according to any one of [5] to [7], wherein the indicator S is $[(T(X_1) - T(X_2))/T(X_3)]$
(wherein $X_1 > X_2$, $X_1 = 20$ to 80, $X_2 = 5$ to 45, and $X_3 = 5$ to 95).
[10] The method according to {3}, wherein

the 3) further comprises estimating the subject blood specimen to be heparin positive when the indicator S is greater than threshold 1 or when the indicator S is equal to or greater than threshold 1,
wherein the threshold 1 is a reference value determined based on indicator S calculated from a blood specimen group including lupus anticoagulant positive.

[11] The method according to [3], wherein

the 3) further comprises estimating the subject blood specimen to be lupus anticoagulant positive when the indicator S is equal to or less than threshold 1 and equal to or greater than threshold 2 or when the indicator S is less than threshold 1 and greater than threshold 2,

wherein the thresholds 1 and 2 are reference values each determined based on indicator S calculated from a blood specimen group including lupus anticoagulant positive.

[12] The method according to [3], wherein

the 3) further comprises estimating the subject blood specimen to be coagulation factor deficiency or coagulation factor inhibitor positive when the indicator S is less than threshold 2 or when the indicator S is equal to or less than threshold 2,

wherein the threshold 2 is a reference value determined based on indicator S calculated from a blood specimen group including lupus anticoagulant positive.

[13] The method according to any one of [10] to [12], wherein the indicator S is $[T(X_1)/T(X_2)]$, (wherein $X_1 < X_2$, $X_1$ = 5 to 20, and $X_2$ = 30 to 85) .

[14] The method according to [7] or [12], wherein the coagulation factor deficiency refers to an FVIII activity value of less than 1%.

[15] The method according to any one of [1] to [14], further comprising calculating coagulation time of the subject blood specimen.

[16] A program for performing the method according to any one of [1] to [15].

[17] An apparatus for performing the method according to any one of [1] to [15].

Advantageous Effects of Invention

According to the method of the present invention, when the coagulation time of a blood specimen is prolonged, a prolongation cause of coagulation time of the blood specimen can be estimated, including a case in which a prolongation cause is heparin.

Brief Description of Drawings

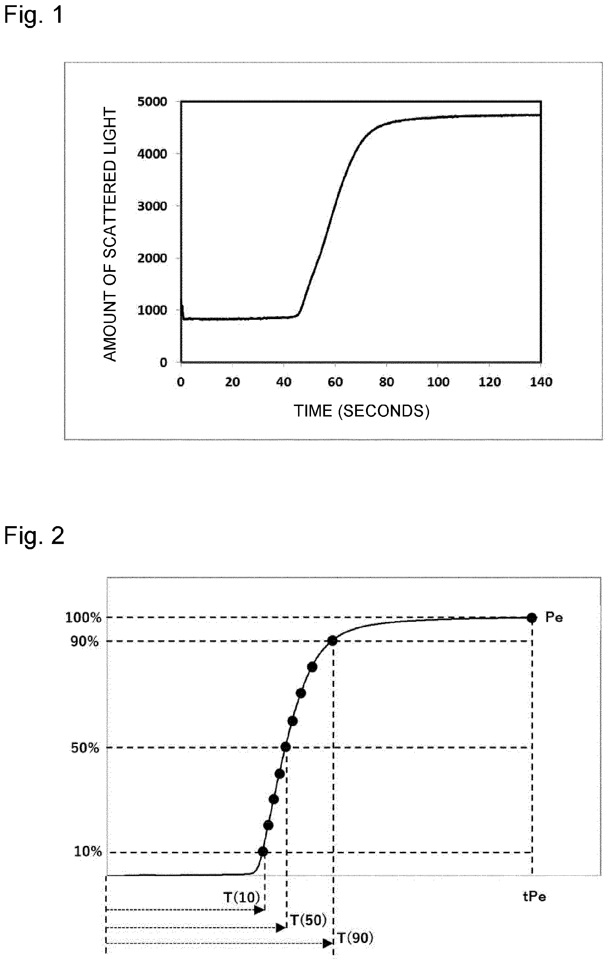

[Fig. 1] Fig. 1 shows an example of a coagulation reaction curve.

[Fig. 2] Fig. 2 describes T(X). The points of time when the coagulation reaction reaches 10%, 50%, and 90% of the Pe on the coagulation reaction curve are indicated by T(10), T(50), and T(90), respectively.

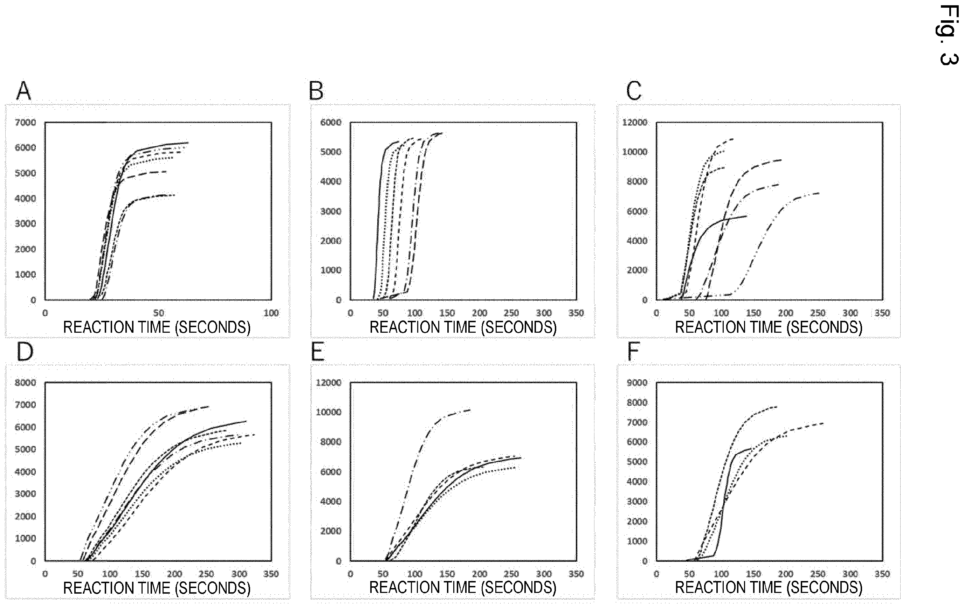

[Fig. 3] Fig. 3 shows coagulation reaction curves of specimens of different specimen types.

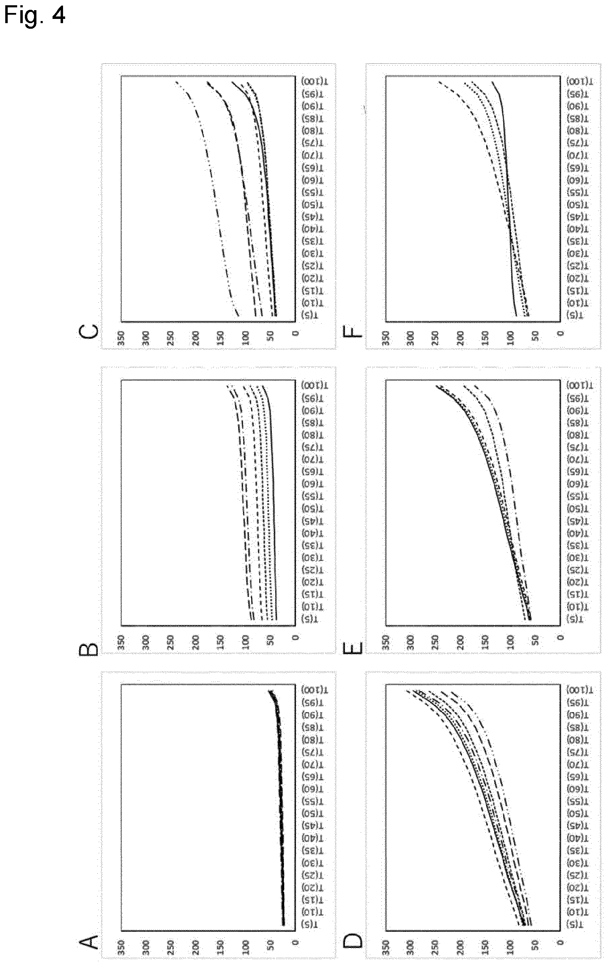

[Fig. 4] Fig. 4 shows T(X)s of specimens of different specimen types.

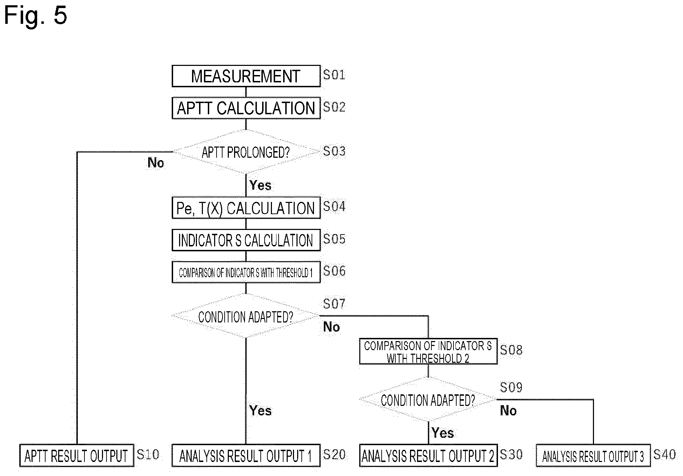

[Fig. 5] Fig. 5 is an exemplary flow for estimating a cause of coagulation time prolongation by the method of the present invention.

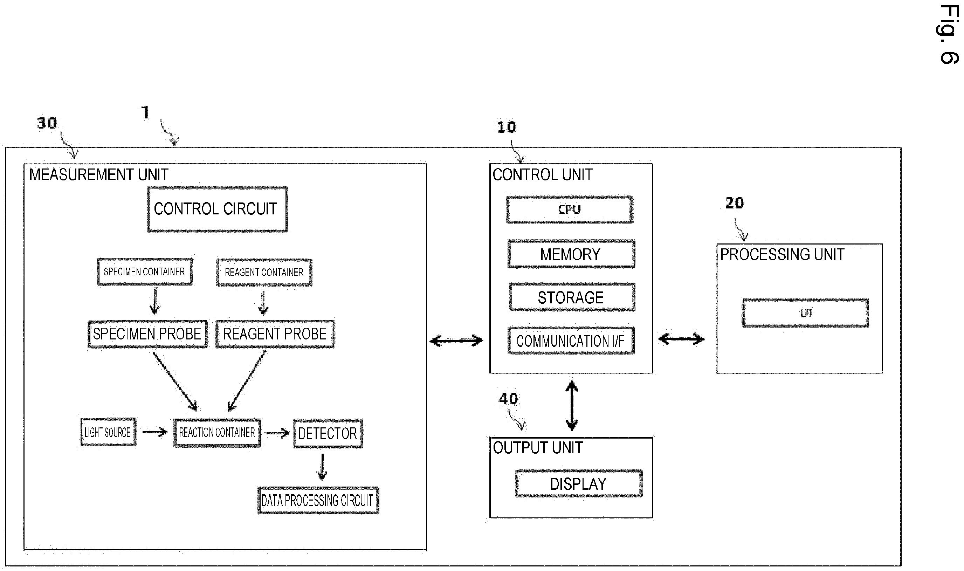

[Fig. 6] Fig. 6 is a conceptual diagram showing a configuration of an automated analyzer for performing a method for estimating a cause of coagulation time prolongation of the present invention.

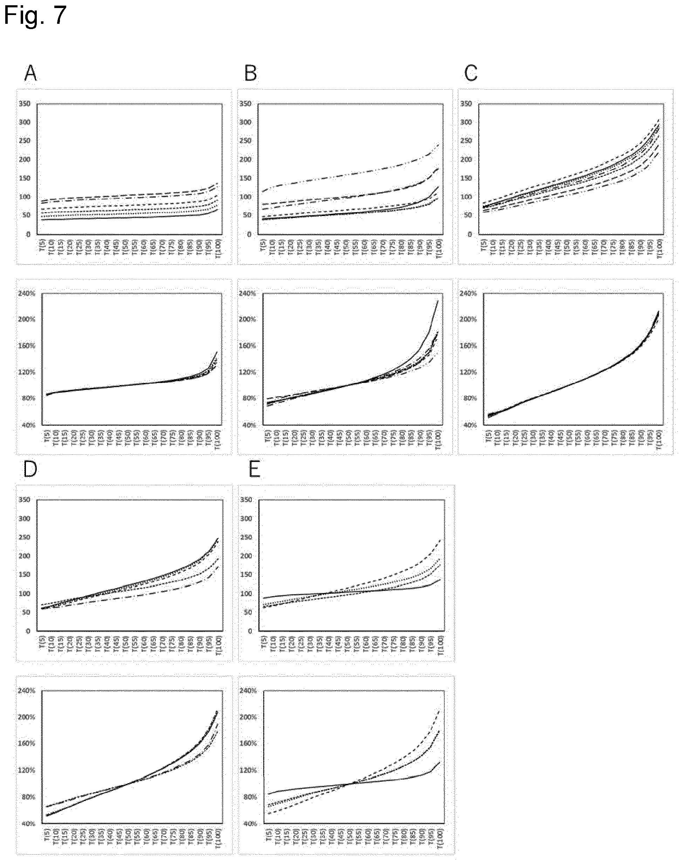

[Fig. 7] Fig. 7 shows absolute values (upper stage) and relative values ($[T(X)/T(50)](\%)$) (lower stage) of T(X) of specimens of different specimen types, i.e., A: heparin, B: LA, C: Inh, D: FVIII, and E: specimen at APTT of about 100 seconds.

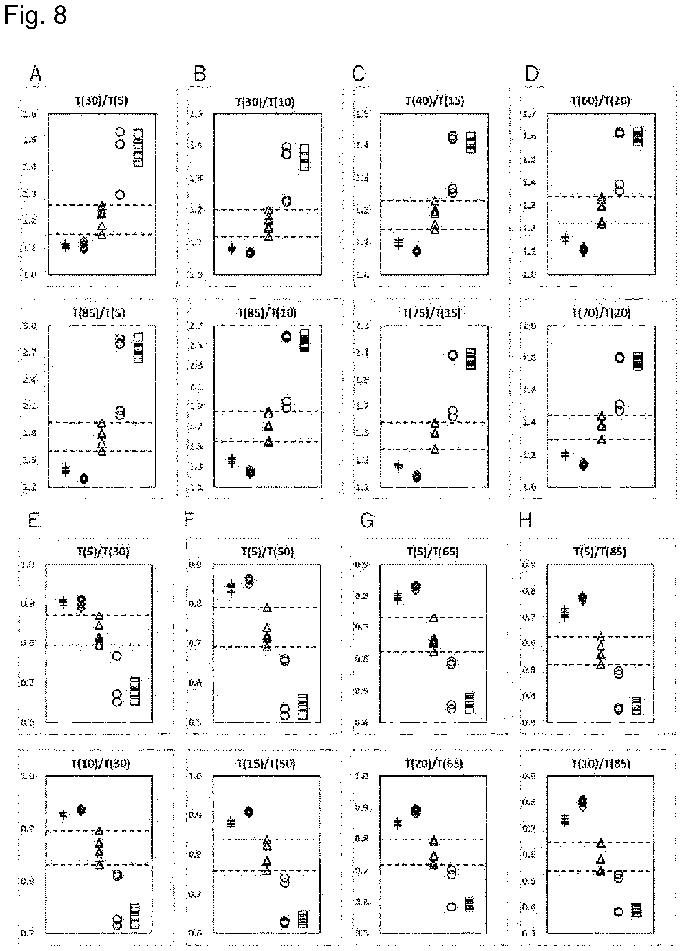

[Fig. 8] Fig. 8 shows plots of examples of $[T(X)/T(5)]$ (A), $[T(X)/T(10)]$ (B), $[T(X)/T(15)]$ (C), $[T(X)/T(20)]$ (D), $[T(X)/T(30)]$ (E), $[T(X)/T(50)]$ (F), $[T(X)/T(65)]$ (G), and $[T(X)/T(85)]$ (H) of different specimen types. The plots in each graph are of NP, heparin, LA, FVIII, and Inh from the left, and different specimen types are represented by different marks. In each graph, the minimum value and the maximum value of the plots of LA are each shown by a dotted line.

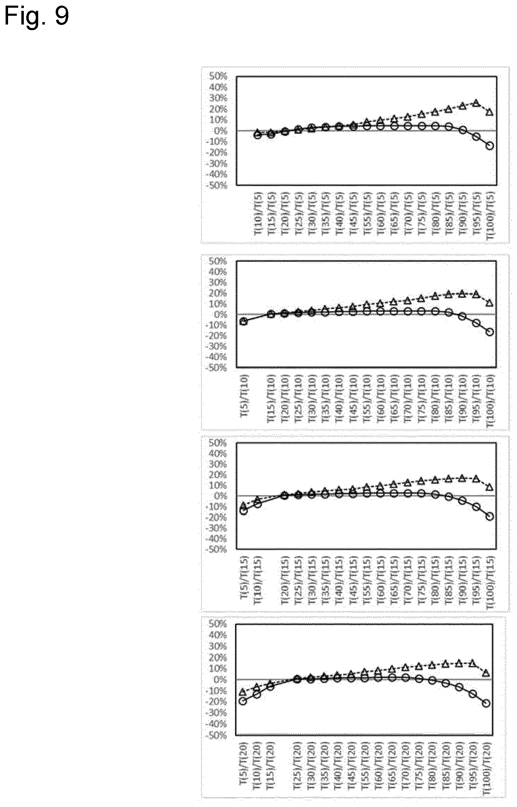

[Fig. 9] Fig. 9 shows changes of the difference (relative value of the difference between the minimum value of one of specimen types and the maximum value of the other) due to the specimen types in $[T(X)/T(5)]$, $[T(X)/T(10)]$, $[T(X)/T(15)]$, and $[T(X)/T(20)]$ from the top. Circle: ratio of difference between the maximum value (a) of LA and the minimum value (b) of FVIII to the average thereof $[(b - a)/\{(b + a)/2\}]$ (LA-FVIII ratio), Triangle: ratio of difference between the maximum value (c) of Heparin and the minimum value (d) of LA to the average thereof $[(d - c)/\{(d + c)/2\}]$ (He-LA ratio).

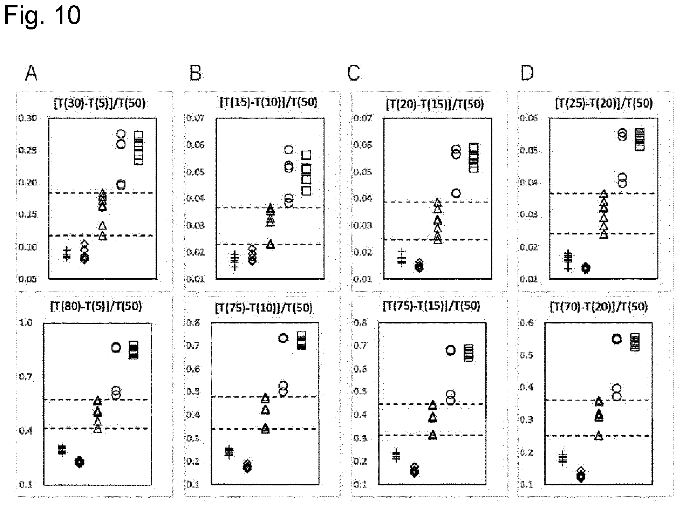

[Fig. 10] Fig. 10 shows plots of examples of $[\{T(X) - T(5)\}/T(50)]$ (A), $[\{T(X) - T(10)\}/T(50)]$(B), $[\{T(X) - T(15)\}/T(50)]$(C), and $[\{T(X) - T(20)\}/T(50)]$ (D) of different specimen types. The plots in each graph are of NP, heparin, LA, FVIII, and Inh from the left, and different specimen types are represented by different marks. In each graph, the minimum value and the maximum value of the plots of LA are each shown by a dotted line.

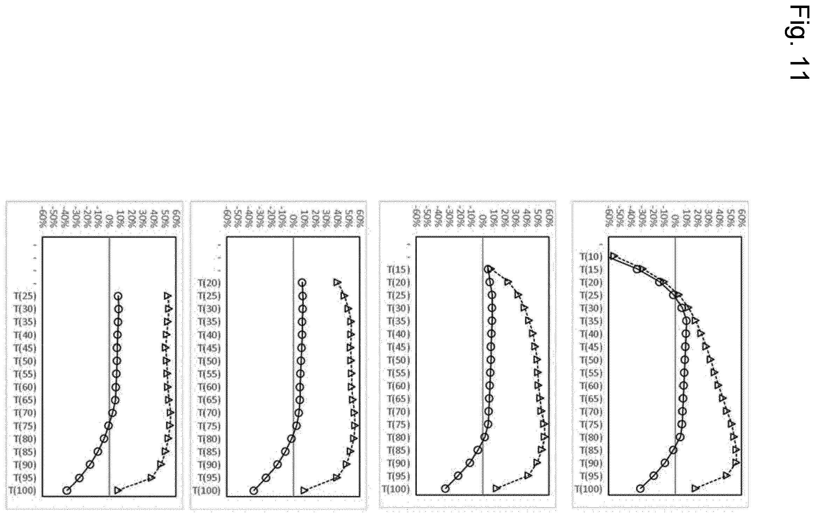

[Fig. 11] Fig. 11 shows changes of the difference (relative value of the difference between the minimum value of one of specimen types and the maximum value of the other) due to the specimen types in [{T(X) - T(5)}/T(50)], [{T(X) - T(10)}/T(50)], [{T(X) - T(15) }/T(50)], and [{T(X) - T(20)}/T(50)] from the top. Circle: ratio of difference between the maximum value (a) of LA and the minimum value (b) of FVIII to the average thereof [(b - a)/{(b + a)/2}] (LA-FVIII ratio), Triangle: ratio of difference between the maximum value (c) of Heparin and the minimum value (d) of LA to the average thereof [(d - c) / { (d + c)/2}] (He-LA ratio).

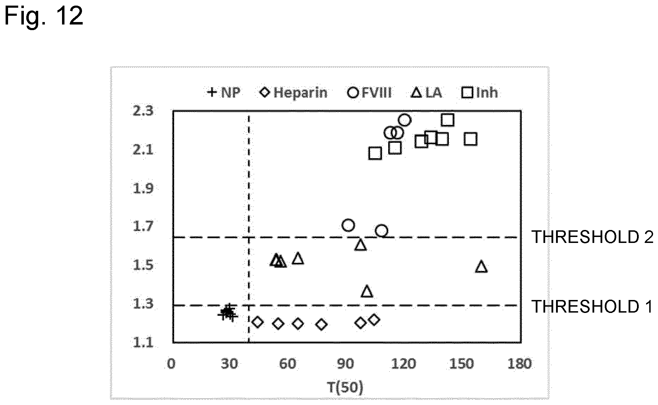

[Fig. 12] Fig. 12 shows an embodiment of estimation of a prolongation cause. T(65)/T(5) is used as the indicator S and plotted with respect to T(50) for each specimen. Threshold 1 for heparin estimation and threshold 2 for LA estimation are each shown by a dotted line. The dotted line parallel to the vertical axis indicates the upper limit of APTT (T(50)) normal range.

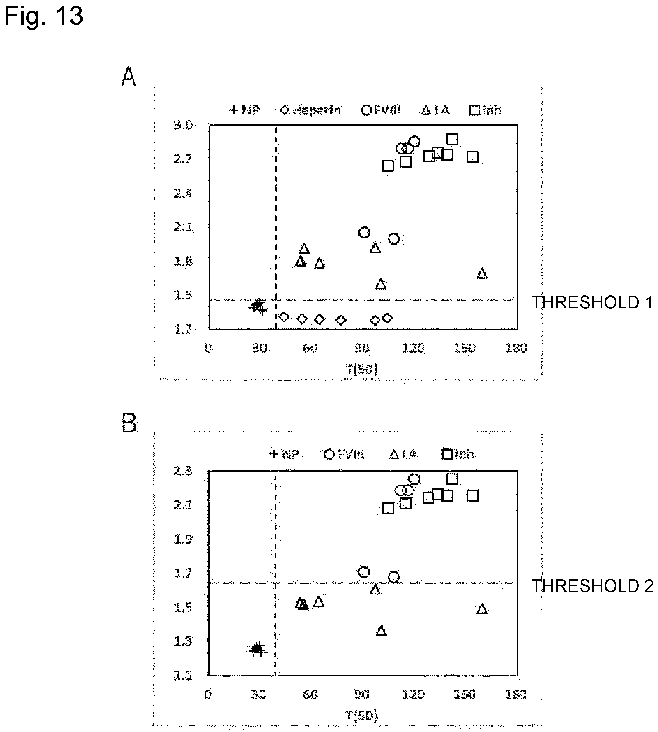

[Fig. 13] Fig. 13 shows an embodiment of estimation of a prolongation cause. Indicator S1 (T(85)/T(5)) and indicator S2 (T(65)/T(5)) are used, and the indicator S1 (A) and the indicator S2 (B) are plotted with respect to T(50) for each specimen. In A of Fig. 13, threshold 1 for heparin estimation is shown by a dotted line. In B of Fig. 13, threshold 2 for LA estimation is shown by a dotted line. In each graph, the dotted line parallel to the vertical axis indicates the upper limit of APTT (T(50)) normal range.

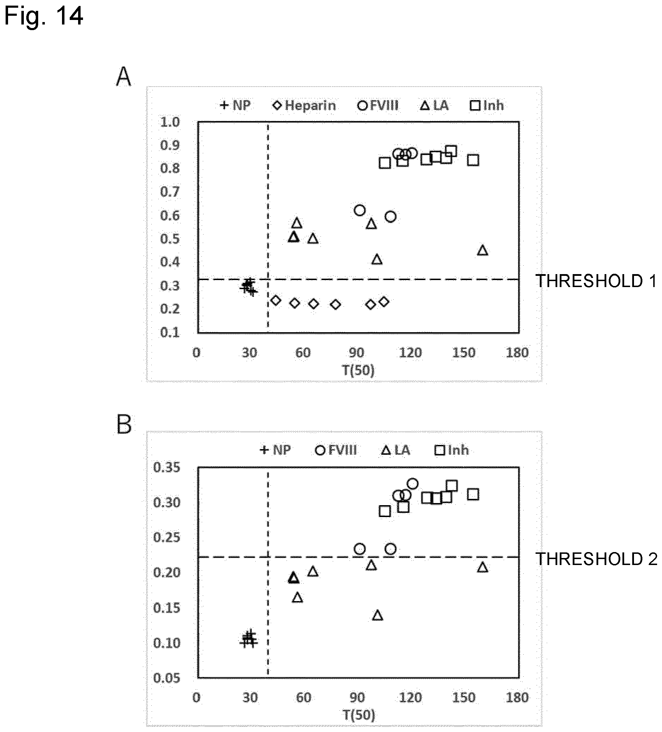

[Fig. 14] Fig. 14 shows an embodiment of estimation of a prolongation cause. Indicator S1 ({T(80) - T(5)}/T(50)) and indicator S2 ({T(35) - T(5)}/T(50)) are used, and the indicator S1 (A) and the indicator S2 (B) are plotted with respect to T(50) for each specimen. In A of Fig. 14, threshold 1 for heparin estimation is shown by a dotted line, and in B of Fig. 14, threshold 2 for LA estimation is shown by a dotted line. In each graph, the dotted line parallel to the vertical axis indicates the upper limit of APTT (T(50)) normal range.

Detailed Description of the Invention

In a blood coagulation test, a predetermined reagent is added to a blood specimen, a subsequent blood coagulation reaction is measured, and the blood coagulation time is measured from the coagulation reaction. In the specification hereinafter, a blood specimen may be referred to as simply a specimen. In the measurement of a blood coagulation reaction, a common means, for example, an optical means that measures the amount of scattered light, transmittance, absorbance, or the like or a mechanical means that measures the viscosity of plasma, is used. The blood coagulation reaction is generally represented by a coagulation reaction curve showing the change with time in the coagulation reaction amount. A coagulation reaction curve based on the amount of scattered light in a normal specimen with no coagulation abnormality, generally, sharply rises, due to progress of coagulation, at the time when a certain amount of time passed since the reagent addition and then reaches plateau as the coagulation reaction approaches the end. In contrast, a coagulation reaction curve of an abnormal specimen with a coagulation abnormality shows various forms depending on the abnormality factor, such as a delay in rise time of the curve and a moderate rise. As a result, in many abnormal specimens, the coagulation time prolongs compared with normal specimens. Prolongation of the coagulation time is an indicator for whether a coagulation abnormality cause is present or not. However, the type of the coagulation abnormality cause (cause of coagulation time prolongation) cannot be estimated from the coagulation time.

When prolongation of coagulation time such as APTT is observed in the blood coagulation test, in general, as a cause for that, contamination of heparin (for example, administration of heparin, blood sampling from a heparinlocked infusion line, or blood sampling after dialysis) is suspected first. A blood specimen contaminated with heparin can be confirmed by that the coagulation time is shortened by protamine sulfate or the like having a heparin-neutralizing effect. When there is no suspicion of heparin contamination in a blood specimen, a cross mixing test is further performed to decide a cause of coagulation time prolongation (hereinafter, also referred to as simply "prolongation cause"). For details, it is judged the prolongation of the APTT is due to which of a coagulation factor inhibitor (anticoagulant), a lupus anticoagulant (LA), and a coagulant factor deficiency such as hemophilia. In the cross mixing test, APTT of normal plasma and test plasma, APTT (immediate reaction) of a plasma mixture of the test plasma and the normal plasma immediately after mixing, and APTT (late reaction) of the plasma mixture after incubation at 37°C for 2 hours are measured. Based on the patterns of these immediate reaction and late reaction, an APTT prolongation cause is judged. In conventional methods, a cross mixing test must be performed separately from coagulation time measurement for judging the prolongation cause. Moreover, since the cross mixing test needs to measure the immediate reaction of a specimen mixture of a subject specimen and a normal specimen and late reaction after incubation for 2 hours, it takes time and effort.

As in Patent Literatures 1 and 2 above, methods for acquiring data relating to a prolongation cause without depending on the cross mixing test have been proposed. In estimation of prolongation causes not depending on the cross mixing test in these conventional technologies, differential curves of a coagulation reaction curve, i.e., coagulation rate (primary differentiation) and coagulation acceleration (secondary differentiation), have been focused on. On the other hand, Patent Literature 3 describes that the time for coagulation reaction to reach a predetermined percentage of the maximum value of a coagulation reaction curve has a relationship with the concentration of the blood coagulation factor. However, since the coagulation reaction curve does not have a peak form unlike a differential curve, parameters based on peak vertexes such as the maximum coagulation rate and the maximum coagulation rate time cannot be obtained. Few attempts have been made to extract information of a prolongation cause from the coagulation reaction curve.

In the present invention, it is possible to estimate a cause of coagulation time prolongation of a specimen by analyzing a waveform of a coagulation reaction curve itself (i.e., zero differentiation) without using a differential curve (primary differentiation or secondary differentiation) of a coagulation reaction curve used in the above-described conventional technologies.

[Method for estimating a cause of coagulation time prolongation]

The present invention provides a method for estimating a cause of coagulation time prolongation. The method for estimating a cause of coagulation time prolongation of the present invention (hereinafter, also referred to as a method of the present invention) uses a blood specimen having a prolonged coagulation time as a subject blood specimen (hereinafter, also referred to as a subject specimen) and estimates a cause of coagulation time prolongation of the subject specimen.

In a common blood coagulation test, time-series data of blood coagulation reaction of a specimen are acquired, and a coagulation reaction curve or coagulation time of the specimen is acquired based on the data. A specimen having a coagulation time longer than a normal value (e.g., standard value determined based on the coagulation time of a normal specimen group) is selected as a specimen having a prolonged coagulation time and can be used as a subject specimen of the method of the present invention.

Examples of the coagulation time to be measured in the present invention include prothrombin time (PT) and activated partial thromboplastin time (APTT). In the specification hereinafter, the method of the present invention will be described mainly taking activated partial thromboplastin time (APTT) as an example of the coagulation time. Modification of the method of the present invention to other coagulation time (e.g., prothrombin time (PT)) can be implemented by those skilled in the art.

The method of the present invention will now be described.

1. Acquisition of coagulation reaction curve

In the method of the present invention, plasma of a subject is preferably used as a blood specimen. An anticoagulant that is commonly used in coagulation tests may be added to the specimen. For example, plasma is obtained by collecting blood using a blood collection tube containing sodium citrate and then performing centrifugation.

In measurement of blood coagulation reaction, a coagulation time measurement reagent is added to a specimen to start the blood coagulation reaction. The coagulation reaction of the mixture liquid including the reagent and the specimen may be measured. The coagulation time measurement reagent to be used can be arbitrarily selected according to the measurement purpose. Various types of reagents for measuring coagulation time are commercially available (e.g., APTT reagent Coagpia APTT-N, manufactured by Sekisui Medical Co., Ltd.). In the measurement of coagulation reaction, a common means, for example, an optical means that measures the amount of scattered light, transmittance, absorbance, or the like or a mechanical means that measures the viscosity of plasma, may be used. In the specification hereinafter, the method of the present invention will be described taking coagulation reaction measurement based on the amount of scattered light as an example.

Although the reaction start point of coagulation reaction may be typically defined as the time when a reagent is mixed with a specimen to start the coagulation reaction, other timing may be defined as the reaction start point. The time of continuing the coagulation reaction measurement may be, for example, from several tens seconds to about seven minutes from the time of mixing a specimen and a reagent. This measurement time may be an arbitrarily determined fixed value or may be time until detection of the end of coagulation reaction of each specimen. During the measurement time, measurement of progress of coagulation reaction (photometry in optical detection) may be performed repeatedly at predetermined intervals. For example, measurement may be performed at 0.1 seconds intervals. The temperature of the mixture liquid during the measurement is a normal condition, for example, 30°C or more and 40°C or less, preferably 35°C or more and 39°C or less. Various conditions for the measurement may be appropriately set according to the specimen, reagent, measurement means, and so on.

A series of operations in the above-described coagulation reaction measurement can be performed using an automated analyzer. One example of the automated analyzer is automated blood coagulation analyzer CP3000 (manufactured by Sekisui Medical Co., Ltd.). Alternatively, a part of operations may be manually performed. For example, preparation of a specimen is performed by a human, and the subsequent operations can be performed with an automated analyzer.

A coagulation reaction curve P(i) is acquired by the coagulation reaction measurement described above. Here, "i" represents the number of measurement points or the time from the start of coagulation reaction (simply also referred to as time). For example, if the measurement (photometry) interval is 0.1 seconds, the time is represented by 0.1 × (the number of measurement points). That is, P(i) may be a function of the number of measurement points or may be a function of the time. In the specification hereinafter, P(i) may be simply abbreviated as P. In general, the coagulation reaction curve P is obtained by subjecting the measurement values of coagulation reaction measurement to noise removal or smoothing process by a usual means or as needed to zero adjustment or relativization of the curve for adjusting the reaction initial value. Fig. 1 shows an example of the coagulation reaction curve. In Fig. 1, the horizontal axis represents time, and the vertical axis represents the amount of scattered light. Since the coagulation reaction of the mixture liquid progresses with time, the amount of scattered light increases.

According to need, in order to calculate the coagulation time or the like, a differential curve, such as a reaction rate curve (primary differentiation) or a reaction acceleration curve (secondary differentiation), of the obtained coagulation reaction curve P may be acquired. The differentiation processing of the coagulation reaction curve can be implemented by an arbitrary method, for example, can be performed by calculation of average inclination value within the interval. In the present specification, a coagulation reaction curve, a primary differential curve such as a reaction rate curve, and a secondary differential curve such as a reaction acceleration curve are also referred to as "zero curve", "primary curve", and "secondary curve", respectively.

1.1. Calculation of coagulation time

The blood coagulation time of a specimen can be calculated based on the coagulation reaction curve P. The coagulation time can be calculated by an arbitrary method. Examples of the method for calculating coagulation time include a method of calculating the time when P(i) reaches N% of the coagulation reaction end point Pe (described later) as coagulation time; a method of calculating the time when the primary curve of P reaches N% of the maximum value as coagulation time; a method of defining the time when the ratio of the integrated value of P(i) in minute time zone reaches a predetermined value as a calculation start point Te and calculating the time when P(i) reaches N% of P(Te) as coagulation time (JP-A-06-249855); a method of calculating coagulation time based on the change with time in the integrated value of P(i) in minute time zone (see JP Application No. 2019-237427); a method of calculating coagulation time based on the weighted average time of a primary curve of P (see JP Application No. 2020-039344); and a method of defining the time when a primary curve of P reaches the maximum value and then reaches a predetermined value as a calculation start point Te and calculating the time when P(i) reaches N% of P(Te) as coagulation time (see JP Application No. 2020-068877) .

When the calculated coagulation time of a specimen is longer than a normal value (e.g., standard value determined based on the coagulation time of a normal specimen group), the coagulation time of the specimen is judged to be prolonged. The specimen having a prolonged coagulation time can be used as a subject specimen of the method of the present invention.

1.2. Detection of coagulation reaction end point Pe

The method of the present invention detects a coagulation reaction end point Pe in a coagulation reaction curve P of a subject specimen. The Pe can be determined according to an arbitrary reference such as the time when P reaches plateau, the time when a primary curve of P reaches the peak and then decreases to 0 or a certain value (see JP Application No. 2020-068877), or the first point at which the ratio of the integrated value of P(i) in minute time zone is less than a threshold (e.g., 1.001) (see JP Application No. 2019-237427 or Example described later). The Pe may be detected after acquisition of P until predetermined measurement time or may be detected simultaneously with acquisition of P and the acquisition of P may be terminated at the time when Pe is detected.

2. Calculation of T(X)

When Pe is detected, T(X) representing the measurement point or time at which the coagulation reaction curve P(i) reaches X% of Pe is calculated. In more detail, T(X) is the number of measurement points or time from the coagulation reaction starting point (time 0) to reaching of P to X% of Pe. That is, T(X) can be calculated by the following expression:

$$P\ (T(X))\ =\ Pe\ \times\ X\%.$$

Here, X is a variable of greater than 0 and equal to or less than 100. Accordingly, T(X) is a function of the variable X. In the method of the present invention, preferably $1 \leq X \leq 99$, and more preferably $5 \leq X \leq 95$. Each X is preferably a value that is 5 or more apart from each other.

Alternatively, T(X) may be represented as an average from T(X-K) to T(X+K). In this case, K is preferably less than the interval between each X. For example, when X = 10 or 15 and K = 2, T(X) when X = 10 is represented as the average from T(8) to T(12), and T(X) when X = 15 is represented as the average from T(13) to T(17).

Fig. 2 is a graph explaining T(X). The graph shows a coagulation reaction curve indicating points at which Pe reaches 10% to 90%, and the points of time when Pe reaches 10%, 50%, and 90% are indicated as T(10), T(50), and T(90), respectively. The point of time tPe of Pe corresponds to T(100).

A to F of Fig. 3 are graphs showing coagulation reaction curves P of normal specimens and specimens of each type of causes of coagulation time prolongation (specimen types). B to E of Fig. 3 show coagulation reaction curves P of each specimen type that prolongs the coagulation time, and coagulation time of these specimens is prolonged compared with that of normal specimens (NP, A of Fig. 3). The forms of coagulation reaction curves P tend to be different depending on the specimen type. In heparin positive (B of Fig. 3), the curves rise sharply. In coagulation factor inhibitor positive (D of Fig. 3) and coagulation factor deficiency (E of Fig. 3), the forms of P are likely to be similar, and all the curves rise relatively gently. The form of P in lupus anticoagulant positive (C of Fig. 3) varies depending on the specimen. F of Fig. 3 shows P of specimens having APTT prolonged to about 100 seconds, and the curves in the graph show respectively different specimen types. Even if the degrees of prolongation of APTT are similar to each other, the form of P varies depending on the specimen type.

A to F of Fig. 4 are graphs showing T(X) when X of each of the same specimens in Fig. 3 is 5 to 100. A to E of Fig. 4 show T(X) of each of the specimen types, and the curves (T(X)) in each graph are of different specimens of the same specimen type. T(X) in each of B to E of Fig. 4 is mostly higher than T(X) of the normal specimens (NP, A of Fig. 4). T(50) that can be defined as coagulation time (APTT) is higher than NP in B to E of Fig. 4, which coincides with the results in Fig. 3. In addition, although the forms of T(X) of normal specimens (NP, A of Fig. 4) are substantially the same, the forms of T(X) of specimen types showing prolonged coagulation time (B to E of Fig. 4) vary among the specimens. F of Fig. 4 shows T(X) of specimens having APTT prolonged to about 100 seconds, and the curves in the graph show respectively different specimen types. Even if the APTT is similar to another, the form of T(X) varies depending on the specimen type.

3. Estimation of cause of coagulation time prolongation based on form of T(X)

In the method of the present invention, the cause of coagulation time prolongation of a subject specimen is estimated based on the form of T(X). In a preferred embodiment, the method of the present invention calculates the indicator S reflecting the form of T(X) of a subject specimen. The cause of coagulation time prolongation of the subject specimen is estimated based on the value of the indicator S.

3.1. Calculation of indicator S

The indicator S may be an arbitrary parameter that can reflect the form of T(X), i.e., T(X) that varies depending on X. In one embodiment, two, three, or four or more Xs different from each other are set for calculating the S.

In one embodiment, two Xs, i.e., $X_1$ and $X_2$, are set. For these two Xs, T(X): $T(X_1)$ and $T(X_2)$ are calculated respectively.

In another embodiment, three Xs, i.e., $X_1$, $X_2$, and $X_3$ are set. For these three Xs, T(X) : $T(X_1)$, $T(X_2)$, and $T(X_3)$ are calculated respectively.

In one embodiment, S can be a relative value of T(X) for an arbitrary X. Preferably, S may be a ratio of $T(X_1)$ and $T(X_2)$, $[T(X_1)/T(X_2)]$ . In this case, $X_1 \neq X_2$ and $X_1 < X_2$ or $X_1 > X_2$. In one embodiment, $X_1 > X_2$, and $X_1$ is preferably 30 to 85, more preferably 60 to 75, and $X_2$ is preferably 5 to 20. In another embodiment, $X_1 < X_2$, and $X_1$ is preferably 5 to 20, more preferably 5 to 15, and $X_2$ is preferably 30 to 85.

In one embodiment, S can be a change rate of T(X) within an arbitrary range of X. Preferably, S can be $[(T(X_1) - T(X_2))/T(X_3)]$. In this case, $X_1 > X_2$ and preferably $X_1 \neq X_3$ and $X_2 \neq X_3$. $X_1$ is preferably 20 to 80, more preferably 25 to 75. $X_2$ is preferably 5 to 45, more preferably 5 to 40. $X_3$ is preferably 5 to 95.

3.2. Estimation of cause of coagulation time prolongation

Examples of the type (specimen type) of the cause of coagulation time prolongation that can be estimated by the method of the present invention include heparin positive (Heparin), lupus anticoagulant positive (LA), coagulation factor deficiency, and coagulation factor inhibitor positive (Inhibitor). Examples of the coagulation factor deficiency include coagulation factor V (FV) deficiency, coagulation factor VIII (FVIII) deficiency, coagulation factor IX (FIX) deficiency, coagulation factor X (FX) deficiency, coagulation factor XI (FXI) deficiency, and coagulation factor XII (FXII) deficiency, and preferred is FVIII deficiency. The FVIII deficiency is preferably a state in which the FVIII activity value is less than 1%. Examples of the inhibitor include an FVIII inhibitor.

The calculated indicator S may exhibit different distributions depending on the specimen type. For example, as shown in Example described later (see Fig. 8), the S of Heparin is smaller or greater than those of other specimen types (e.g., LA, coagulation factor deficiency, and inhibitor). The S of LA is likely to be greater than those of normal specimen and Heparin but to be less than those of coagulation factor deficiency and Inhibitor, or is likely to be less than those of normal specimen and Heparin but to be greater than those of coagulation factor deficiency and Inhibitor. Accordingly, the indicator S can be used for estimating the cause of coagulation time prolongation (specimen type) of a subject specimen. That is, the cause of coagulation time prolongation of a subject specimen can be estimated based on the indicator S.

In one embodiment, the cause of coagulation time prolongation of a subject blood specimen is estimated by comparing the indicator S of the subject specimen with a reference value. The reference value can be determined prior to the implementation of the method of the present invention. The reference value can be determined based on the indicator S of a specimen group in which the cause of coagulation time prolongation is known.

In one example, it is possible to calculate the indicator S of each specimen from a specimen group in which the cause of coagulation time prolongation is known (specimens include each of Heparin, LA, Inhibitor, and coagulation factor deficiency and may include a normal specimen) and to determine a reference value for discriminating a specimen having a specific prolongation cause (heparin, LA, inhibitor, or coagulation factor deficiency) to be estimated from specimens having other prolongation causes based on the calculated indicator S.

In another example, it is possible to calculate the indicator S of each specimen from a specimen group having a specific prolongation cause (heparin, LA, inhibitor, or coagulation factor deficiency) and to determine a reference value for discriminating a specimen having the specific prolongation cause from specimens having other prolongation causes based on the calculated indicator S.

For example, the S of Heparin is smaller or greater than those of other specimen types (LA, Inhibitor, and coagulation factor deficiency). In one embodiment, when $S = [T(X_1)/T(X_2)]$ and $X_1 > X_2$ (preferably, $X_1 = 30$ to $85$, more preferably $60$ to $75$, and $X_2 = 5$ to $20$), the S of Heparin is less than those of other specimen types. In another embodiment, when $S = [T(X_1)/T(X_2)]$ and $X_1 < X_2$ (preferably, $X_1 = 5$ to $20$, more preferably $5$ to $15$, and $X_2 = 30$ to $85$), the S of Heparin is greater than those of other specimen types. Furthermore, in another embodiment, when $S = [(T(X_1) - T(X_2))/T(X_3)]$ (wherein $X_1 > X_2$, preferably $X_1 \neq X_3$ and $X_2 \neq X_3$, $X_1$ is preferably $20$ to $80$, more preferably $25$ to $75$, $X_2$ is preferably $5$ to $45$, more preferably $5$ to $40$, and $X_3$ is preferably $5$ to $95$), the S of Heparin is less than those of other specimen types.

Accordingly, in one embodiment, a reference value of an indicator S for distinguishing Heparin from other specimen types (LA, Inhibitor, and coagulation factor deficiency) is set. The reference value can be determined based on the indicator S of a specimen group including various specimen types (including specimens of Heparin, LA, Inhibitor, and coagulation factor deficiency). Alternatively, the reference value can be determined based on the indicator S of a specimen group including Heparin and LA. Alternatively, the reference value can be determined based on the indicator S of a specimen group including Heparin only.

More specifically, the reference value for detecting Heparin may be set as a value (threshold 1) that can distinguish between the indicator S of Heparin and the indicator S of LA in a specimen group. For example, regarding an indicator S of Heparin being less than those of other specimen types, the threshold 1 may be set between the maximum value of the S of Heparin and the minimum value of the S of LA in the specimen group. If the indicator S of a subject specimen is equal to or less than the threshold 1 or less than the threshold 1, the subject specimen can be estimated as Heparin. In contrast, regarding an indicator S of Heparin being greater than those of other specimen types, the threshold 1 may be set between the minimum value of the S of Heparin and the maximum value of the S of LA in the specimen group. When the indicator S of a subject specimen is greater than or equal to the threshold 1 or greater than the threshold 1, the subject specimen can be estimated as Heparin.

In addition, for example, regarding an indicator S of Heparin being less than those of other specimen types, the S of LA is greater than those of a normal specimen and Heparin and is less than those of coagulation factor deficiency and Inhibitor specimens. Alternatively, regarding and indicator S of Heparin being greater than those of other specimen types, the S of LA is less than those of a normal specimen and Heparin and greater than those of coagulation factor deficiency and Inhibitor specimens. Accordingly, in another embodiment, the reference value of the indicator S for distinguishing between Heparin and LA or the reference value of the indicator S for distinguishing LA from Inhibitor and coagulation factor deficiency is set. The reference value can be determined based on the indicator S of a specimen group including various specimen types (including specimens of Heparin, LA, Inhibitor, and coagulation factor deficiency). Alternatively, the reference value can be determined based on the indicator S of a specimen group including LA only.

Preferably, a threshold 1 for distinguishing between LA and Heparin and a threshold 2 for distinguishing LA from Inhibitor and coagulation factor deficiency are determined as reference values. For example, regarding an indicator S of LA being greater than those of normal specimen and Heparin and being less than those of coagulation factor deficiency and Inhibitor specimens, the threshold 1 may be set between the maximum value of the S of Heparin and the minimum value of the S of LA in the specimen group, and the threshold 2 may be set between the maximum value of the S of LA and the minimum value of the S of Inhibitor and coagulation factor deficiency. When the indicator S of a subject specimen is less than the threshold 1, the subject specimen can be estimated as Heparin. When the indicator S of a subject specimen is equal to or greater than the threshold 1 and equal to or less than the threshold 2, the subject specimen can be estimated as LA. When the indicator S of a subject specimen is greater than the threshold 2, the subject specimen can be estimated as Inhibitor or coagulation factor deficiency. Alternatively, regarding the same indicator S, when the indicator S of a subject specimen is equal to or less than the threshold 1, the subject specimen can be estimated as Heparin. When the indicator S of a subject specimen is greater than the threshold 1 and less than the threshold 2, the subject specimen can be estimated as LA. When the indicator S of a subject specimen is equal to or greater than the threshold 2, the subject specimen can be estimated as Inhibitor or coagulation factor deficiency.

In contrast, regarding an indicator S of which the value of LA is less than those of normal specimen and Heparin and is greater than those of coagulation factor deficiency and Inhibitor specimens, a threshold 1 may be set between the minimum value of the S of Heparin and the maximum value of the S of LA, and a threshold 2 may be set between the minimum value of the S of LA and the maximum value of the S of Inhibitor and coagulation factor deficiency in the specimen group. When the indicator S of a subject specimen is greater than the threshold 1, the subject specimen can be estimated as Heparin. When the indicator S of a subject specimen is equal to or less than the threshold 1 and equal to or greater than the threshold 2, the subject specimen can be estimated as LA. When the indicator S of a subject specimen is less than the threshold 2, the subject specimen can be estimated as Inhibitor or coagulation factor deficiency. Alternatively, regarding the same indicator S, when the indicator S of a subject specimen is equal to or greater than the threshold 1, the subject specimen can be estimated as Heparin. When the indicator S of a subject specimen is less than the threshold 1 and greater than the threshold 2, the subject specimen can be estimated as LA. When the indicator S of a subject specimen is equal to or less than the threshold 2, the subject specimen can be estimated as Inhibitor or coagulation factor deficiency.

Those skilled in the art will understand that as long as different specimen types can be differentiated and detected by the estimation criteria of specimen types above, the reference values of "less than threshold 1 (or threshold 2)" and "equal to or less than threshold 1 (or threshold 2)" are exchangeable and the reference values of "greater than threshold 1 (or threshold 2)" and "equal to or greater than threshold 1 (or threshold 2)" are exchangeable.

In order to estimate one specimen type, two or more indicators S may be used, or two or more reference values for one indicator S may be used. For example, when two indicators S of a subject specimen both satisfy reference values, the specimen type can be estimated. For example, when two indicators S of a subject specimen both satisfy reference values, the conformance is defined as ++, and when only one of the indicators S satisfies the reference value, the conformance is defined as +. Thus, the accuracy of the estimation of a specimen type can be evaluated. Alternatively, when an indicator S of a subject specimen satisfies both of two reference values, the conformance is defined as ++, and when the indicator S satisfies only one of the reference values satisfies, the conformance is defined as +. Thus, the accuracy of the estimation of a specimen type can be evaluated.

The estimation result of the prolongation cause (specimen type) of a subject specimen can be output by an arbitrary format. As needed, information such as the coagulation time and the accuracy of the estimation can be output together with the estimation result of the specimen type of a subject specimen. The estimation result of the specimen type of a subject specimen and the coagulation time both are preferably output.

As a preferable embodiment, Fig. 5 shows an exemplary flow for estimating the cause of coagulation time prolongation of a subject specimen by the method of the present invention. The detailed procedure of the flow of Fig. 5 is shown below.

S01: Coagulation reaction of a subject specimen (e.g., plasma) as a measurement sample is measured.

S02: APTT is calculated from the measurement of S01.

S03: When the APTT calculated in S02 is a normal value (within reference range), the step proceeds to S10, and when the APTT is an abnormal value (prolongation), the step proceeds to S04.

S04: Pe and T(X) are calculated using the coagulation reaction curve P(i) obtained by the measurement in S01.

S05: Indicator S is calculated using T(X) calculated in S04.

S06: The indicator S is compared with threshold 1 for estimating the specimen type.

S07: When the specimen type can be estimated in S06, the step proceeds to S20, and when the specimen type cannot be estimated, the step proceeds to S08.

S08: The indicator S is compared with threshold 2 for estimating the specimen type.

S09: When the specimen type can be estimated in S08, the step proceeds to S30, and when the specimen type cannot be estimated, the step proceeds to S40.

S10: APTT is output (APTT is normal).

S20: APTT and estimated specimen type are output.

S30: APTT and estimated specimen type are output.

S40: APTT and the result showing that the specimen type cannot be estimated are output.

4. Application to other coagulation reaction measurement method

The method of estimating a cause of coagulation time prolongation of the present invention has been described using coagulation reaction measurement based on the amount of scattered light. However, those skilled in the art can apply the method based on the amount of scattered light to other coagulation reaction measuring methods (e.g., blood coagulation reaction measuring methods based on transmittance, absorbance, viscosity, or the like), and consequently such applications are encompassed in the scope of the present invention.

5. Program and apparatus

The above-described method for estimating a cause of coagulation time prolongation of the present invention may be automatically performed using a computer program. Accordingly, one aspect of the present invention is a program for performing the above-described method for estimating a cause of coagulation time prolongation of the present invention. In a preferable embodiment, the program of the present invention is a program for implementing the flow of Fig. 5 described above. A series of processes of the method of the present invention described above can be automatically performed by an automated analyzer. Accordingly, one aspect of the present invention is an apparatus for performing the method for estimating a cause of coagulation time prolongation of the present invention described above.

One embodiment of the apparatus of the present invention will now be described. One embodiment of the apparatus of the present invention is an automated analyzer 1 as shown in Fig. 6. The automated analyzer 1 includes a control unit 10, an operation unit 20, a measurement unit 30, and an output unit 40.

The control unit 10 controls the whole operation of the automated analyzer 1. The control unit 10 may be constituted of, for example, a personal computer (PC). The control unit 10 includes a CPU, a memory, a storage, a communication interface (I/F), and so on and performs processing of the commands from the operation unit 20, control of the operation of the measurement unit 30, storage and data analysis of measurement data received from the measurement unit 30, storage of analysis result, control of output of the measurement data and analysis results by the output unit 40, and so on. The control unit 10 may be further connected to other equipment such as an external medium and a host computer. In the control unit 10, the PC that controls the operation of the measurement unit 30 and the PC that analyzes measurement data may be the same or different.

The operation unit 20 acquires the input from an operator and transmits the obtained input information to the control unit 10. For example, the operation unit 20 includes a user interface (UI) such as a key board or a touch panel. The output unit 40 outputs analysis results such as the measurement data of the measurement unit 30 and the coagulation reaction curve P, coagulation reaction end point Pe, coagulation time, T(X), indicator S, and the estimation result of a cause of coagulation time prolongation of a specimen (e.g., heparin, LA, or other than them) based on the measurement data under the control by the control unit 10. For example, the output unit 40 includes a display apparatus such as a display.

The measurement unit 30 implements a series of operations for a blood coagulation test and acquires measurement data of coagulation reaction of samples including a blood specimen. The measurement unit 30 includes various types of equipment and analysis modules necessary for a blood coagulation test, for example, a specimen container for containing a blood specimen, a reagent container for containing a test reagent, a reaction container for a reaction of the specimen and the reagent, a probe for dispensing the blood specimen and the reagent to the reaction container, a light source, a detector for detecting scattered light or transmitted light from the sample in the reaction container, a data processing circuit for sending data from the detector to the control unit 10, and a control circuit for controlling the operation of the measurement unit 30 according to the command from the control unit 10.

The control unit 10 estimates the cause of coagulation time prolongation of a specimen based on data measured by the measurement unit 30. This analysis may include acquisition of the above-described coagulation reaction curve P and coagulation time, detection of the coagulation reaction end point Pe, calculation of T(X) and indicator S, and so on. The control unit 10 may further perform estimation of the cause of coagulation time prolongation of a specimen using the calculated S. Alternatively, the coagulation reaction curve P and Pe may be acquired by the control unit 10 based on the measurement data from the measurement unit 30 or may be acquired by another equipment, for example, the measurement unit 30 and may be sent to the control unit 10. The control unit 10 may store the coagulation time of a specimen and various parameters to be used for calculation of Pe, T(X), and S (e.g., equations for calculating T(X) and S). The control unit 10 may store reference values (e.g., the above-described thresholds 1 and 2) to be used for estimating the cause of coagulation time prolongation of a specimen. Alternatively, the control unit 10 may incorporate the parameters and reference values stored in external equipment or on a network at the time of analysis.

The analysis above can be implemented by a program for performing the method of the present invention. Accordingly, the control unit 10 may include a program for performing the method for estimating a cause of coagulation time prolongation of the present invention.

The analysis result in the control unit 10 is sent to the output unit 40 and is output. The output can be in an arbitrary form such as display on a screen, sending to a host computer, or printing. The output information from the output unit may include data of a coagulation reaction curve, the coagulation reaction end point Pe, the coagulation time, the T(X), the indicator S, estimation result of a cause of coagulation time prolongation of a specimen, and so on. The type of the output information from the output unit may be controlled by a program of the present invention.

Examples

The present invention will now be described in further detail by Examples but is not limited to these Examples.

Example 1

1. Method

1.1) Specimen

As normal specimens (NP), three types of pooled plasma containing citrate (each N = 1) obtained from normal subjects, Coagpia calibrator N of Sekisui Medical Co., Ltd. (N = 1), Coagtrol N of Sysmex Corporation (N = 1), pooled normal plasma of George King Bio-Medical, Inc. (N = 1), and CRYOcheck Pooled Normal Plasma of Precision BioLogic Inc. (N = 1) were used (total, N = 7).
As FVIII deficient plasma (FVIII), Factor VIII Deficient of George King Bio-Medical, Inc. confirmed to have a FVIII activity value of less than 1% was used (N = 1 in each of five lots, Total N = 5).
As LA positive specimens (LA), Positive Lupus Anticoagulant Plasma of George King Bio-Medical, Inc. was used (N = 1 in each of seven lots, Total N = 7).
As FVIII inhibitor positive specimens (Inh), Factor VIII Deficient with Inhibitor of George King Bio-Medical, Inc. was used (N = 1 in each of six lots, Total N = 6).
As heparin-containing specimens (Heparin), heparin concentration series specimens of six levels were prepared such that the heparin concentrations were from 0.3 U/mL to 0.8 U/mL with 0.1 U/mL intervals by adding heparin (Heparin Na Injection 5000 U/5 mL "Mochida" of Mochida Pharmaceutical Co., Ltd.) to pooled plasma containing citrate obtained from normal subjects (N = 1 at each concentration, Total N = 6).

1.2) Coagulation reaction measurement

As measurement reagents, Coagpia APTT-N (manufactured by Sekisui Medical Co., Ltd.) was used as the APTT measurement reagent, and Coagpia APTT-N calcium chloride liquid (manufactured by Sekisui Medical Co., Ltd.) was used as the calcium chloride liquid. The coagulation reaction measurement of samples including a specimen was performed using an automated blood coagulation analyzer CP3000 (manufactured by Sekisui Medical Co., Ltd.). A specimen (50 $\mu$L) was heated at 37°C for 45 seconds in a cuvette, a reagent for measurement (50 $\mu$L) of about 37°C was then added thereto, and after 171 seconds, 25 mM calcium chloride liquid (50 $\mu$L) was further added thereto to start coagulation reaction. The reaction was performed at 37°C. In the measurement of the coagulation reaction, the cuvette was irradiated with light of a wavelength of 660 nm from LED as a light source, and the amount of scattered light of the 90-degree side scattered light was measured at intervals of 0.1 seconds. The measurement time was set to 360 seconds.

2. Analysis of coagulation reaction curve

2.1) Acquisition of coagulation reaction curve

The photometry data of each specimen were subjected to a smoothing process including noise removal, and zero adjustment process was then performed such that the amount of scattered light at the photometry stating point was 0 to produce a coagulation reaction curve P(i).
2.2) Acquisition of coagulation reaction end point Pe and determination of APTT
The P(i) at the time when the integration ratio Z(i) of the coagulation reaction curve P(i) (see JP Application No. 2019-237427) was less than the integration ratio threshold (1.001) at the first time was detected as the coagulation reaction end point Pe. The time T(50) at which P(i) reached 50% of Pe was calculated and was determined as APTT. The integration ratio Z(i) at the time i was calculated as follows.

$$\text{Integration ratio } Z(i) = Pb(i)/Pa(i)$$

$$Pa(i) = the\ sum\ from\ P(i-20)\ to\ P(i-1)$$

$$Pb(i) = the\ sum\ from\ P(i+1)\ to\ P(i+20)$$

The range of APTT of each specimen type is shown below. When APTT exceeded 39 seconds, it was judged that the APTT was prolonged.

NP: 26.1 to 30.8 seconds
FVIII: 91.1 to 119.7 seconds
LA: 53.2 to 159.3 seconds
Inh: 115.9 to 154.6 seconds
Heparin: 43.5 to 104.1 seconds

As described above, the APTT of any of four specimen types other than NP was prolonged, and the ranges thereof overlapped. For example, the ranges of APTT of the four specimen types other than NP all included near 100 seconds. These results demonstrated that specimen types cannot be discriminated by APTT only and that another analysis is necessary for discriminating these specimen types.

At the same time, as shown in Fig. 3, the coagulation reaction curve P varies depending on the specimen type. In Heparin (B of Fig. 3), the APTT is prolonged than that of NP (A of Fig. 3), and the rise of the curve of P is steep as in NP. In Inh (D of Fig. 3) and FVIII (E of Fig. 3), the forms of P tend to be similar to each other, and the rise of both curves of P are relatively gentle. The form of P of LA (C of Fig. 3) varies depending of the specimen and is partially similar to that of Heparin and partially similar to Inh or FVIII. As shown in F of Fig. 3, even if APTT approximated near 100 seconds, the form of P varied depending on the specimen type.

2.3) Calculation of T(X)

The time T(X) at which the coagulation reaction curve P(i) reaches X% of the Pe detected in section 2.2) was calculated by the following expression. Twenty Xs were set from 5 to 100 in increments of 5, and T(5) to T(100) were calculated. The T(50) at X = 50 corresponds to APTT.

$$P (T(X)) = Pe \times X\%$$

As shown in Fig. 4, in each T(X) of Heparin, LA, Inh, and FVIII (B to E of Fig. 4), APTT was prolonged and was therefore greater than NP (A of Fig. 4). In addition, although the forms of T(X) of NP were approximately the same, the forms of T(X) of other four specimen types (B to E of Fig. 4) varied depending on the specimen type. As shown in F of Fig. 4, even if the APTT approximated near 100 seconds, the form of T(X) varied depending on the specimen type.

Fig. 7 shows the absolute values (same as B to F of Fig. 4) of T(X) in the upper stage and the relative values ([T(X)/T(50)](%)) of T(X) in the lower stage of Heparin, LA, Inh, and FVIII (A to D of Fig. 7) and specimens of specimen types of which each APTT was near 100 seconds (E of Fig. 7). It was demonstrated that the degree of overlapping of the relative value curves and the height of the left end vary depending on the specimen type.

A to D of Fig. 8 are graphs of plotting examples of [T(X)/T(5)], [T(X)/T(10)], [T(X)/T(15)], and [T(X)/T(20)] of each specimen type. The plots in each graph represent NP, Heparin, LA, FVIII, and Inh from the left, and the different specimen types are indicated by different marks. In each graph, the minimum value and the maximum value of the plots for LA are shown by dotted lines, and these dotted lines divided plots of LA from the plots of Heparin and from the plots of FVIII and Inh. It was demonstrated that Heparin, LA, and FVIII/Inh can be divided based on the relative values of T(X) with respect to T(5) to T(20) . E to H of Fig. 8 are graphs of plotting examples of [T(X)/T(30)], [T(X)/T(50)], [T(X)/T(65)], and [T(X)/T(85)] of each specimen type. The meanings of the marks of plots and dotted lines in each graph are the same as those in A to D of Fig. 8. It was demonstrated that Heparin, LA, and FVIII/Inh can be divided based on the relative values of T(X) with respect to T(30) to T(85).

Fig. 9 shows changes of the difference (relative value of the difference between the minimum value of one of specimen types and the maximum value of the other) due to the specimen types in [T(X)/T(5)], [T(X)/T(10)], [T(X)/T(15)], and [T(X)/T(20)] from the top (X = 5 to 100). In the graphs, the circle is the ratio of the difference between the maximum value (a) of LA and the minimum value (b) of FVIII to the average thereof [(b - a)/{(b + a)/2}] (LA-FVIII ratio), and the triangle is the ratio of the difference between the maximum value (c) of Heparin and the minimum value (d) of LA to the average thereof [(d - c) / { (d + c)/2}] (He-LA ratio).

The LA-FVIII ratio was greater than 2% in the follows:

T(X)/T(5): 30 to 85;
T(X)/T(10): 30 to 85;
T(X)/T(15): 40 to 75; and
T(X)/T(20): 60 to 70.

The He-LA ratio was greater than 2% in the follows:

T(X)/T(5): 30 to 100;
T(X)/T(10): 25 to 100;
T(X)/T(15): 25 to 100; and
T(X)/T(20): 30 to 100.

The results of LA-FVIII ratio and He-LA ratio are collectively shown in Table 1.

[Table 1]

| Indicator | LA-FVIII ratio | | | He-LA ratio | | |
|---|---|---|---|---|---|---|
| | Range of X greater than 2% | X at the maximum | Maximum | Range of X greater than 2% | X at the maximum | Maximum |
| T(X)/T(5) | 30-85 | 65 | 4.6% | 30-100 | 95 | 25.5% |
| T(X)/T(10) | 30-85 | 65 | 3.4% | 25-100 | 90 | 19.5% |
| T(X)/T(15) | 40-75 | 65 | 2.7% | 25-100 | 90 | 16.9% |
| T(X)/T(20) | 60-70 | 65 | 2.2% | 30-100 | 90 | 15.0% |

A to D of Fig. 10 are graphs of plotting examples of [{T(X) - T(5)}/T(50)], [{T(X) - T(10)}/T(50)], [{T(X) - T(15)}/T(50)], and [{T(X) - T(20)}/T(50)] of each specimen type. The plots in each graph represent NP, Heparin, LA, FVIII, and Inh from the left, and the different specimen types are indicated by different marks. In each graph, the minimum value and the maximum value of the plots for LA are shown by dotted lines respectively, and these dotted lines divided plots of LA from the plots of Heparin and from the plots of FVIII and Inh. It was demonstrated that Heparin, LA, and FVIII/Inh can be divided based on the change rate of T(X).

Fig. 11 shows changes of the difference (relative value of the difference between the minimum value of one of specimen types and the maximum value of the other) due to the specimen types in [{T(X) - T(5)}/T(50)], [{T(X) - T(10)}/T(50)], [{T(X) - T(15)}/T(50)], and [{T(X) - T(20)}/T(50)] from the top (X = 5 to 100). In the graphs, the circle is the ratio of the difference between the maximum value (a) of LA and the minimum value (b) of FVIII to the average thereof [(b - a)/{(b + a)/2}] (LA-FVIII ratio), and the triangle is the ratio of the difference between the maximum value (c) of Heparin and the minimum value (d) of LA to the average thereof [(d - c)/{(d + c)/2}] (He-LA ratio) .

The LA-FVIII ratio was greater than 2% in the follows:

[{T(X)-T(5)}/T(50)]: 30 to 80;
[{T(X)-T(10)}/T(50)]: 15 to 75;
[{T(X)-T(15)}/T(50)]: 20 to 75; and
[{T(X)-T(20)}/T(50)]: 25 to 70.

The He-LA ratio was greater than 2% in the follows:

[{T(X)-T(5)}/T(50)]: 25 to 100;
[{T(X)-T(10)}/T(50)]: 15 to 100;
[{T(X)-T(15)}/T(50)]: 20 to 100; and
[{T(X)-T(20)}/T(50)]: 25 to 100.

The results of LA-FVIII ratio and He-LA ratio are collectively shown in Table 2.

[Table 2]

| Indicator | LA-FVIII ratio | | | He-LA ratio | | |
|---|---|---|---|---|---|---|
| | Range of X greater than 2% | X at the maximum | Maximum | Range of X greater than 2% | X at the maximum | Maximum |
| [T(X)-T(5)]/T(50) | 30-80 | 35 | 10.3% | 25-100 | 85 | 55.3% |
| [T(X)-T(10)]/T(50) | 15-75 | 25-30 | 8.9% | 15-100 | 80 | 56.3% |
| [T(X)-T(15)]/T(50) | 20-75 | 30 | 8.8% | 20-100 | 75 | 56.1% |
| [T(X)-T(20)]/T(50) | 25-70 | 30 | 8.7% | 25-100 | 70 | 55.2% |

3. Estimation of prolongation cause

3.1) Estimation using one indicator S (T(X) relative value)

As the indicator S, T(65)/T(5) at which the LA-FVIII ratio was the maximum value was used. Fig. 12 shows plots of indicator S with respect to T(50) in each specimen. In the graph, the dotted line parallel to the vertical axis represents the upper limit of APTT (T(50)) normal range, and specimens exceeding this are in the state of APTT prolongation. The lower line of two dotted lines parallel to the horizontal axis represents the average of the maximum value of the S of Heparin and the minimum value of the S of LA, which was defined as a threshold 1, and the upper line represents the average of the maximum value of the S of LA and the minimum value of the S of FVIII, which was defined as a threshold 2. In APTT prolonged specimens, a specimen of which the indicator S was less than the threshold 1 was estimated as Heparin. In the remaining specimens, a specimen of which the indicator S was less than the threshold 2 was estimated as LA, and a specimen of which the indicator S was equal to or greater than the threshold 2 was estimated as FVIII or Inh. As shown in Fig. 12, in the specimens of LA, although the APTT of the specimen of which the APTT was most prolonged was greater than those of FVIII and Inh, the indicator S was less than those of FVIII and Inh. This result demonstrated that the value of indicator S does not depend on APTT (T(50)).

3.2) Estimation using two indicators S (T(X) relative values)

As an indicator S1 for estimating Heparin, T(85)/T(5) was used. As an indicator S2 for estimating LA, T(65)/T(5) was used. A of Fig. 13 is plots of the indicator S1 with respect to T(50) of each specimen. In the graph, the dotted line parallel to the vertical axis represents the upper limit of APTT (T(50)) normal range. The dotted line parallel to the horizontal axis represents the average of the maximum value of the S of Heparin and the minimum value of the S of LA, which was defined as a threshold 1. In the APTT prolonged specimens, a specimen of which the indicator S1 is less than the threshold 1 was estimated as Heparin. B of Fig. 13 is plots of the indicator S2 with respect to T(50) of each specimen. Heparin specimens are not displayed. In the graph, the dotted line parallel to the vertical axis represents the upper limit of APTT (T(50)) normal range. The dotted line parallel to the horizontal axis represents the average of the maximum value of the S of LA and the minimum value of the S of FVIII, which was defined as a threshold 2. In the APTT prolonged specimens, a specimen of which the indicator S2 was the threshold 2 was estimated as LA, and a specimen of which the indicator S2 was equal to or greater than the threshold 2 was estimated as FVIII or Inh.

3.3) Estimation using two indicators S (T(X) change rates)

As an indicator S1 for estimating Heparin, {T(80) - T(5) }/T(50) was used. As an indicator S2 for estimating LA, {T(35) - T(5) }/T(50) was used. A of Fig. 14 is plots of the indicator S1 with respect to T(50) of each specimen. In the graph, the dotted line parallel to the vertical axis represents the upper limit of APTT (T(50)) normal range. The dotted line parallel to the horizontal axis represents the average of the maximum value of the S of Heparin and the minimum value of the S of LA, which was defined as a threshold 1. In APTT prolonged specimens, a specimen of which the indicator S1 is less than the threshold 1 was estimated as Heparin. B of Fig. 14 is plots of the indicator S2 with respect to T(50) of each specimen. Specimens of Heparin are not displayed. In the graph, the dotted line parallel to the vertical axis represents the upper limit of the APTT (T(50)) normal range. The dotted line parallel to the horizontal axis represents the average of the maximum value of the S of LA and the minimum value of the S of FVIII, which was defined as a threshold 2. In APTT prolonged specimens, a specimen of which the indicator S2 was less than the threshold 2 was estimated as LA, and a specimen of which the indicator S2 was equal to or greater than the threshold 2 was estimated as FVIII or Inh.

**Claims**

1.  A method for estimating a cause of coagulation time prolongation, comprising:

    1) detecting a coagulation reaction end point Pe in a coagulation reaction curve of a subject blood specimen having a prolonged coagulation time;
    2) calculating T(X), wherein T(X) represents a measurement point or time at which the coagulation reaction curve reaches X% of Pe, and X is a variable of greater than 0 and equal to or less than 100; and
    3) estimating a cause of coagulation time prolongation of the subject blood specimen based on a form of T(X).

2.  The method according to claim 1, wherein the 3) comprises calculating an indicator S representing the form of T(X) and estimating a cause of coagulation time prolongation of the subject blood specimen based on a value of the indicator S.

3.  The method according to claim 2, wherein the indicator S is an indicator representing a relative value of T(X) or a change rate of T(X).

4.  The method according to claim 3, wherein

    the 3) further comprises estimating a cause of coagulation time prolongation of the subject blood specimen by comparing the indicator S with a reference value, and
    the reference value is determined based on an indicator S calculated from a blood specimen group in which the cause of coagulation time prolongation is known.

5.  The method according to claim 3, wherein

    the 3) further comprises estimating the subject blood specimen to be heparin positive when the indicator S is less than threshold 1 or when the indicator S is equal to or less than threshold 1,
    wherein the threshold 1 is a reference value determined based on indicator S calculated from a blood specimen group including lupus anticoagulant positive.

6.  The method according to claim 3, wherein

    the 3) further comprises estimating the subject blood specimen to be lupus anticoagulant positive when the indicator S is equal to or greater than threshold 1 and equal to or less than threshold 2 or when the indicator S is greater than threshold 1 and less than threshold 2,
    wherein the thresholds 1 and 2 are reference values each determined based on indicator S calculated from a blood specimen group including lupus anticoagulant positive.

7.  The method according to claim 3, wherein

    the 3) further comprises estimating the subject blood specimen to be coagulation factor deficiency or coagulation factor inhibitor positive when the indicator S is greater than threshold 2 or when the indicator S is equal to or greater than threshold 2,
    wherein the threshold 2 is a reference value determined based on indicator S calculated from a blood specimen group including lupus anticoagulant positive.

8.  The method according to any one of claims 5 to 7, wherein the indicator S is $[T(X_1)/T(X_2)]$, wherein $X_1 > X_2$, $X_1 = 30$ to $85$, and $X_2 = 5$ to $20$.

9. The method according to any one of claims 5 to 7, wherein the indicator S is $[(T(X_1) - T(X_2))/T(X_3)]$, wherein $X_1 > X_2$, $X_1 = 20$ to $80$, $X_2 = 5$ to $45$, and $X_3 = 5$ to $95$.

10. The method according to claim 3, wherein

   the 3) further comprises estimating the subject blood specimen to be heparin positive when the indicator S is greater than threshold 1 or when the indicator S is equal to or greater than threshold 1,
   wherein the threshold 1 is a reference value determined based on indicator S calculated from a blood specimen group including lupus anticoagulant positive.

11. The method according to claim 3, wherein

   the 3) further comprises estimating the subject blood specimen to be lupus anticoagulant positive when the indicator S is equal to or less than threshold 1 and equal to or greater than threshold 2 or when the indicator S is less than threshold 1 and greater than threshold 2,
   wherein the thresholds 1 and 2 are reference values each determined based on indicator S calculated from a blood specimen group including lupus anticoagulant positive.

12. The method according to claim 3, wherein

   the 3) further comprises estimating the subject blood specimen to be coagulation factor deficiency or coagulation factor inhibitor positive when the indicator S is less than threshold 2 or when the indicator S is equal to or less than threshold 2,
   wherein the threshold 2 is a reference value determined based on indicator S calculated from a blood specimen group including lupus anticoagulant positive.

13. The method according to any one of claims 10 to 12, wherein the indicator S is $[T(X_1)/T(X_2)]$, wherein $X_1 < X_2$, $X_1 = 5$ to $20$, and $X_2 = 30$ to $85$.

14. The method according to claim 7 or 12, wherein the coagulation factor deficiency refers to an FVIII activity value of less than 1%.

15. The method according to any one of claims 1 to 14, further comprising calculating coagulation time of the subject blood specimen.

16. A program for performing the method according to any one of claims 1 to 15.

17. An apparatus for performing the method according to any one of claims 1 to 15.

Fig. 1

Fig. 2

Fig. 3

Fig. 4

Fig. 5

```
                    ┌──────────────────┐
                    │  MEASUREMENT     │ S01
                    └──────────────────┘
                    ┌──────────────────┐
                    │ APTT CALCULATION │ S02
                    └──────────────────┘

                      ╱ APTT PROLONGED? ╲  S03
              No    ╱                     ╲
                    ╲                     ╱
                      ╲                 ╱
                           │ Yes
                    ┌──────────────────┐
                    │ Pe, T(X) CALCULATION │ S04
                    └──────────────────┘
                    ┌──────────────────┐
                    │ INDICATOR S CALCULATION │ S05
                    └──────────────────┘
                    ┌──────────────────────────────────┐
                    │ COMPARISON OF INDICATOR S WITH THRESHOLD 1 │ S06
                    └──────────────────────────────────┘
                                              S07
                      ╱ CONDITION ADAPTED? ╲
                    ╲                       ╱ No
                      ╲                   ╱
                                                    ┌──────────────────────────┐
                                                    │ COMPARISON OF INDICATOR S │ S08
                                                    │    WITH THRESHOLD 2       │
                                                    └──────────────────────────┘
                                                                  S09
                                                      ╱ CONDITION ADAPTED? ╲
                                                    ╲                       ╱ No
                                                      ╲                   ╱
                           │ Yes                             │ Yes
```

┌────────────────────┐  ┌──────────────────────────┐  ┌──────────────────────────┐  ┌──────────────────────────┐
│ APTT RESULT OUTPUT │ S10 │ ANALYSIS RESULT OUTPUT 1 │ S20 │ ANALYSIS RESULT OUTPUT 2 │ S30 │ ANALYSIS RESULT OUTPUT 3 │ S40
└────────────────────┘  └──────────────────────────┘  └──────────────────────────┘  └──────────────────────────┘

Fig. 6

Fig. 7

# Fig. 8

Fig. 9

Fig. 10

Fig. 11

Fig. 12

Fig. 13

A

THRESHOLD 1

B

THRESHOLD 2

Fig. 14

A

B

## INTERNATIONAL SEARCH REPORT

| International application No. |
|---|
| **PCT/JP2022/009429** |

### A. CLASSIFICATION OF SUBJECT MATTER

**G01N 33/86**(2006.01)i
FI:   G01N33/86

According to International Patent Classification (IPC) or to both national classification and IPC

### B. FIELDS SEARCHED

Minimum documentation searched (classification system followed by classification symbols)

G01N33/86

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2020/158948 A1 (SEKISUI MEDICAL CO LTD) 06 August 2020 (2020-08-06) claims | 1-3, 15-17 |
| A | JP 2019-86517 A (SEKISUI MEDICAL CO LTD) 06 June 2019 (2019-06-06) entire text, all drawings | 1-17 |
| A | WADA, Hideo et al. Update on the Clot Waveform Analysis. Clinical and Applied Thrombosis/Hemostasis. 30 August 2020, vol. 26, article no. 1076029620912027 entire text, all drawings | 1-17 |
| A | SHIMOMURA, Daiki et al. The First-Derivative Curve of the Coagulation Waveform Reveals the Cause of aPTT Prolongation. Clinical and Applied Thrombosis/Hemostasis. 29 December 2020, vol. 26, article no. 1076029620978810 entire text, all drawings | 1-17 |
| A | WO 2020/256107 A1 (SEKISUI MEDICAL CO LTD) 24 December 2020 (2020-12-24) entire text, all drawings | 1-17 |
| A | WO 2020/101025 A1 (SEKISUI MEDICAL CO LTD) 22 May 2020 (2020-05-22) entire text, all drawings | 1-17 |

| ✓ Further documents are listed in the continuation of Box C. | ✓ See patent family annex. |
|---|---|

| * Special categories of cited documents: |
|---|
| "A" document defining the general state of the art which is not considered to be of particular relevance |
| "E" earlier application or patent but published on or after the international filing date |
| "L" document which may throw doubts on priority claim(s) or which is cited to establish the publication date of another citation or other special reason (as specified) |
| "O" document referring to an oral disclosure, use, exhibition or other means |
| "P" document published prior to the international filing date but later than the priority date claimed |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2022/009429** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2002-541431 A (AKZO NOBEL N.V) 03 December 2002 (2002-12-03)<br>entire text, all drawings | 1-17 |

Form PCT/ISA/210 (second sheet) (January 2015)

**INTERNATIONAL SEARCH REPORT**
Information on patent family members

International application No.

**PCT/JP2022/009429**

| Patent document cited in search report | | | Publication date (day/month/year) | Patent family member(s) | | | Publication date (day/month/year) |
|---|---|---|---|---|---|---|---|
| WO | 2020/158948 | A1 | 06 August 2020 | (Family: none) | | | |
| JP | 2019-86517 | A | 06 June 2019 | (Family: none) | | | |
| WO | 2020/256107 | A1 | 24 December 2020 | JP entire text, all drawings | 2021-1884 | A | |
| WO | 2020/101025 | A1 | 22 May 2020 | CN whole document | 113039436 | A | |
| JP | 2002-541431 | A | 03 December 2002 | US whole document | 2003/0049851 | A1 | |
| | | | | WO | 2000/046603 | A1 | |
| | | | | EP | 1147423 | A1 | |
| | | | | CA | 2362055 | A1 | |
| | | | | AU | 3483300 | A | |
| | | | | KR | 10-2002-0021811 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016194426 A
- JP 2016118442 A
- WO 2020101025 A
- JP 6249855 A
- JP 2019237427 A
- JP 2020039344 A
- JP 2020068877 A